(19) Europäisches Patentamt
European Patent Office
Office européen des brevets

(11) **EP 3 202 912 A1**

(12) **EUROPEAN PATENT APPLICATION**
published in accordance with Art. 153(4) EPC

(43) Date of publication:
09.08.2017 Bulletin 2017/32

(51) Int Cl.:
*C12Q 1/68* (2006.01)   *C12M 1/34* (2006.01)
*C12N 15/09* (2006.01)

(21) Application number: 15846525.2

(22) Date of filing: 28.09.2015

(86) International application number:
PCT/JP2015/077328

(87) International publication number:
WO 2016/052405 (07.04.2016 Gazette 2016/14)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA

(30) Priority: 29.09.2014 JP 2014199417

(71) Applicant: Fujifilm Corporation
Minato-ku
Tokyo 106-8620 (JP)

(72) Inventors:
• ISHII, Yasuyuki
Ashigara-kami-gun
Kanagawa 258-8538 (JP)
• INOUE, Yuki
Ashigara-kami-gun
Kanagawa 258-8538 (JP)
• OUCHI, Aya
Ashigara-kami-gun
Kanagawa 258-8538 (JP)

(74) Representative: Klunker IP
Patentanwälte PartG mbB
Destouchesstraße 68
80796 München (DE)

(54) **NONINVASIVE METHOD AND SYSTEM FOR DETERMINING FETAL CHROMOSOMAL ANEUPLOIDY**

(57) The present invention provides a noninvasive discrimination method of chromosomal heteroploidy of a fetus, the method including a step of analyzing DNA of a candidate cell of a nucleated red blood cell isolated from maternal blood, in which the fetuses are fetuses classified into any one case selected from monotocous, monochorionic monoamniotic twin fetuses, monochorionic diamniotic twin fetuses, and dichorionic diamniotic twin fetuses based on results of ultrasonic inspections and the number of candidate cells of nucleated red blood cells to be used for analyzing the DNA is optimized based on the classification, and a noninvasive discrimination system of chromosomal heteroploidy of a fetus.

FIG. 1

EP 3 202 912 A1

SEQUENCE LISTING

<110> Fujifilm Corporation

<120> Method of non-invasive discrimination of fetus chromosomal
      aneuploidy and  system for non-invasive discrimination of fetus
      chromosomal aneuploidy

<130> W-5427PCT

<150> JP2014-199417
<151> 2014-09-29

<160> 53

<170> PatentIn version 3.5

<210> 1
<211> 37
<212> DNA
<213> Artificial Sequence

<220>
<223> SeqID_1: rs2737699-F

<400> 1
cgctcttccg atctctgtgc agtcatcttg ctctaca                               37


<210> 2
<211> 37
<212> DNA
<213> Artificial Sequence

<220>
<223> SeqID_2: rs2737699-R

<400> 2
cgctcttccg atctgaccag ttttgatggc tccgaag                               37


<210> 3
<211> 37
<212> DNA
<213> Artificial Sequence

<220>
<223> SeqID_3: rs9552929-F

<400> 3
cgctcttccg atctctgccg cacaacaggt aaaagac                               37


<210> 4
<211> 37
<212> DNA
<213> Artificial Sequence

<220>
<223> SeqID_4: rs9552929-R

<400> 4
cgctcttccg atctgacacg gtatttccct ggttctg                               37

```
<210>   5
<211>   37
<212>   DNA
<213>   Artificial Sequence

<220>
<223>   SeqID_5: rs3751355-F

<400>   5
cgctcttccg atctctgggg aataagggtg acgtggg                          37


<210>   6
<211>   37
<212>   DNA
<213>   Artificial Sequence

<220>
<223>   SeqID_6: rs3751355-R

<400>   6
cgctcttccg atctgaccca gcctcttatt ccccgga                          37


<210>   7
<211>   37
<212>   DNA
<213>   Artificial Sequence

<220>
<223>   SeqID_7: rs3751357-F

<400>   7
cgctcttccg atctctgtgc tttcactgtg gggctca                          37


<210>   8
<211>   37
<212>   DNA
<213>   Artificial Sequence

<220>
<223>   SeqID_8: rs3751357-R

<400>   8
cgctcttccg atctgacata gaccccagca atgtggc                          37


<210>   9
<211>   37
<212>   DNA
<213>   Artificial Sequence

<220>
<223>   SeqID_9: rs202198487-F

<400>   9
cgctcttccg atctctgggc aacattgggc ctttggg                          37


<210>   10
```

```
<211>   37
<212>   DNA
<213>   Artificial Sequence

<220>
<223>   SeqID_10: rs202198487-R

<400>   10
cgctcttccg atctgaccct cgatctcctt tccagcc                                    37


<210>   11
<211>   37
<212>   DNA
<213>   Artificial Sequence

<220>
<223>   SeqID_11: rs2230162-F

<400>   11
cgctcttccg atctctgtgt ccatgcaaac cctggaa                                    37


<210>   12
<211>   37
<212>   DNA
<213>   Artificial Sequence

<220>
<223>   SeqID_12: rs2230162-R

<400>   12
cgctcttccg atctgacaag tggggagagt ggctgtt                                    37


<210>   13
<211>   37
<212>   DNA
<213>   Artificial Sequence

<220>
<223>   SeqID_13: rs3745051-F

<400>   13
cgctcttccg atctctggcc attgagaaag gctgctc                                    37


<210>   14
<211>   37
<212>   DNA
<213>   Artificial Sequence

<220>
<223>   SeqID_14: rs3745051-R

<400>   14
cgctcttccg atctgaccaa gggcacagtc tttgggg                                    37


<210>   15
<211>   37
<212>   DNA
<213>   Artificial Sequence
```

```
<220>
<223>   SeqID_15: rs581894-F


<400>   15
cgctcttccg atctctgcat ctccaggctg tgcagtg                            37



<210>   16
<211>   37
<212>   DNA
<213>   Artificial Sequence


<220>
<223>   SeqID_16: rs581894-R


<400>   16
cgctcttccg atctgacggt ccctttccca caagcag                            37



<210>   17
<211>   37
<212>   DNA
<213>   Artificial Sequence


<220>
<223>   SeqID_17: rs8096037-F


<400>   17
cgctcttccg atctctggca gcaggaggaa ggcaaaa                            37



<210>   18
<211>   37
<212>   DNA
<213>   Artificial Sequence


<220>
<223>   SeqID_18: rs8096037-R


<400>   18
cgctcttccg atctgactcc caagcctgac catacct                            37



<210>   19
<211>   37
<212>   DNA
<213>   Artificial Sequence


<220>
<223>   SeqID_19: rs7234309-F


<400>   19
cgctcttccg atctctggca acatgacaag cactggg                            37



<210>   20
<211>   37
<212>   DNA
<213>   Artificial Sequence


<220>
<223>   SeqID_20: rs7234309-R
```

```
<400>  20
cgctcttccg atctgaccgc tactgggact ggctgat                                    37


<210>  21
<211>  37
<212>  DNA
<213>  Artificial Sequence

<220>
<223>  SeqID_21: rs9305426-F

<400>  21
cgctcttccg atctctgctg tggttatggc ggctgtg                                    37


<210>  22
<211>  37
<212>  DNA
<213>  Artificial Sequence

<220>
<223>  SeqID_22: rs9305426-R

<400>  22
cgctcttccg atctgacagg agtgggagac atagcca                                    37


<210>  23
<211>  37
<212>  DNA
<213>  Artificial Sequence

<220>
<223>  SeqID_23: rs12053674-F

<400>  23
cgctcttccg atctctgagc cacagccagt tccatag                                    37


<210>  24
<211>  37
<212>  DNA
<213>  Artificial Sequence

<220>
<223>  SeqID_24: rs12053674-R

<400>  24
cgctcttccg atctgacctc ctgaaatcat gtgttgc                                    37


<210>  25
<211>  37
<212>  DNA
<213>  Artificial Sequence

<220>
<223>  SeqID_25: rs10775648-F

<400>  25
cgctcttccg atctctgctt cgagtctgtg gatcgcg                                    37
```

```
<210>  26
<211>  37
<212>  DNA
<213>  Artificial Sequence

<220>
<223>  SeqID_26: rs10775648-R

<400>  26
cgctcttccg atctgacgca aaggagtatg gagaggc                     37


<210>  27
<211>  37
<212>  DNA
<213>  Artificial Sequence

<220>
<223>  SeqID_27: rs10775648-F2

<400>  27
cgctcttccg atctctgctt cgagtctgtg gatcgcg                     37


<210>  28
<211>  37
<212>  DNA
<213>  Artificial Sequence

<220>
<223>  SeqID_28: rs10775648-R2

<400>  28
cgctcttccg atctgacgca aaggagtatg gagaggc                     37


<210>  29
<211>  37
<212>  DNA
<213>  Artificial Sequence

<220>
<223>  SeqID_29: rs2073370-F

<400>  29
cgctcttccg atctctggcc agacatgtgc ctcgaag                     37


<210>  30
<211>  37
<212>  DNA
<213>  Artificial Sequence

<220>
<223>  SeqID_30: rs2073370-R

<400>  30
cgctcttccg atctgactcg tgcagcagaa gacactt                     37


<210>  31
```

```
<211>   37
<212>   DNA
<213>   Artificial Sequence

<220>
<223>   SeqID_31: rs457705-F

<400>   31
cgctcttccg atctctggcc caagaccaac tgtgtcc                       37


<210>   32
<211>   37
<212>   DNA
<213>   Artificial Sequence

<220>
<223>   SeqID_32: rs457705-R

<400>   32
cgctcttccg atctgaccaa ggacgactgg ctgttcc                       37


<210>   33
<211>   37
<212>   DNA
<213>   Artificial Sequence

<220>
<223>   SeqID_33: COL18A1-F

<400>   33
cgctcttccg atctctgcag caaagccaca gtccttc                       37


<210>   34
<211>   37
<212>   DNA
<213>   Artificial Sequence

<220>
<223>   SeqID_34: COL18A1-R

<400>   34
cgctcttccg atctgacccc caccctgaca tccttac                       37


<210>   35
<211>   37
<212>   DNA
<213>   Artificial Sequence

<220>
<223>   SeqID_35: TBL1X-F

<400>   35
cgctcttccg atctctgctg ttttgggtgg gtttggt                       37


<210>   36
<211>   37
<212>   DNA
<213>   Artificial Sequence
```

```
<220>
<223>  SeqID_36: TBL1X-R

<400>  36
cgctcttccg atctgacatt tgaaagtggg gcaccat                              37


<210>  37
<211>  37
<212>  DNA
<213>  Artificial Sequence

<220>
<223>  SeqID_37: CTPS2-F

<400>  37
cgctcttccg atctctgggt acttctggga tgccttg                              37


<210>  38
<211>  37
<212>  DNA
<213>  Artificial Sequence

<220>
<223>  SeqID_38: CTPS2-R

<400>  38
cgctcttccg atctgacgtt gcagactaag gcccacc                              37


<210>  39
<211>  37
<212>  DNA
<213>  Artificial Sequence

<220>
<223>  SeqID_39: PHF16-F

<400>  39
cgctcttccg atctctgtgt tggtcttgtt cttgcgt                              37


<210>  40
<211>  37
<212>  DNA
<213>  Artificial Sequence

<220>
<223>  SeqID_40: PHF16-R

<400>  40
cgctcttccg atctgacgac ccatctgtga cgctgtg                              37


<210>  41
<211>  37
<212>  DNA
<213>  Artificial Sequence

<220>
<223>  SeqID_41: ELK1-F
```

```
<400>  41
cgctcttccg atctctgggg cccagggagt catacat                              37


<210>  42
<211>  37
<212>  DNA
<213>  Artificial Sequence

<220>
<223>  SeqID_42: ELK1-R

<400>  42
cgctcttccg atctgactca cggccttctt ctccagg                              37


<210>  43
<211>  37
<212>  DNA
<213>  Artificial Sequence

<220>
<223>  SeqID_43: TMEM185A-F

<400>  43
cgctcttccg atctctgaca cacactgccc atggaac                              37


<210>  44
<211>  37
<212>  DNA
<213>  Artificial Sequence

<220>
<223>  SeqID_44: TMEM185A-R

<400>  44
cgctcttccg atctgaccaa ctcagccaag cccagtc                              37


<210>  45
<211>  37
<212>  DNA
<213>  Artificial Sequence

<220>
<223>  SeqID_45: CY_2655422-674_S-F

<400>  45
cgctcttccg atctctgaga ccacacgatg aatgcgt                              37


<210>  46
<211>  37
<212>  DNA
<213>  Artificial Sequence

<220>
<223>  SeqID_46: CY_2655422-674_S-R

<400>  46
cgctcttccg atctgacggc aacgtccagg atagagt                              37
```

```
<210>  47
<211>  69
<212>  DNA
<213>  Artificial Sequence

<220>
<223>  SeqID_47: D501-F

<400>  47
aatgatacgg cgaccaccga gatctacact atagccttct ttccctacac gacgctcttc      60

cgatctctg                                                              69


<210>  48
<211>  69
<212>  DNA
<213>  Artificial Sequence

<220>
<223>  SeqID_48: D701-R

<400>  48
caagcagaag acggcatacg agatcgagta atgtgactgg agttcagacg tgtgctcttc      60

cgatctgac                                                              69


<210>  49
<211>  69
<212>  DNA
<213>  Artificial Sequence

<220>
<223>  SeqID_49: D702-R

<400>  49
caagcagaag acggcatacg agattctccg gagtgactgg agttcagacg tgtgctcttc      60

cgatctgac                                                              69


<210>  50
<211>  69
<212>  DNA
<213>  Artificial Sequence

<220>
<223>  SeqID_50: D703-R

<400>  50
caagcagaag acggcatacg agataatgag cggtgactgg agttcagacg tgtgctcttc      60

cgatctgac                                                              69


<210>  51
<211>  69
<212>  DNA
<213>  Artificial Sequence
```

```
<220>
<223>   SeqID_51: D704-R

<400>   51
caagcagaag acggcatacg agatggaatc tcgtgactgg agttcagacg tgtgctcttc          60

cgatctgac                                                                  69


<210>   52
<211>   69
<212>   DNA
<213>   Artificial Sequence

<220>
<223>   SeqID_52: D705-R

<400>   52
caagcagaag acggcatacg agatttctga atgtgactgg agttcagacg tgtgctcttc          60

cgatctgac                                                                  69


<210>   53
<211>   69
<212>   DNA
<213>   Artificial Sequence

<220>
<223>   SeqID_53: D706-R

<400>   53
caagcagaag acggcatacg agatacgaat tcgtgactgg agttcagacg tgtgctcttc          60

cgatctgac                                                                  69
```

**Description**

**BACKGROUND OF THE INVENTION**

1. Field of the Invention

The present invention relates to a noninvasive discrimination method and a noninvasive discrimination system or a noninvasive discrimination device of chromosomal heteroploidy of a fetus. More specifically, the present invention relates to a noninvasive discrimination method and a noninvasive discrimination system or a noninvasive discrimination device of chromosomal heteroploidy of a fetus performed through DNA analysis of a candidate cell of a nucleated red blood cell isolated from maternal blood.

2. Description of the Related Art

In the related art, methods for checking chromosomes or genes after collecting fetal cells through an amniocentesis or villus collection have been performed as prenatal diagnosis in order to diagnose a chromosomal abnormality or a genetic abnormality of a fetus. However, a significant problem in that there is a slight risk of complications such as miscarriage due to specimen collection is pointed out in these methods.

In recent years, it is known that fetal cells are transferred into maternal blood and circulate in the mother together with mother-derived blood cells. It is possible to realize safe prenatal diagnosis in which there is no possibility of miscarriage, through reliably reproducibly analyzing DNA of such a chromosome of a fetal cell in maternal blood. However, it is understood that there is only about one fetal cell (fetal nucleated red blood cell), which exists in maternal blood, in the number (mL) of maternal blood. Therefore, reliable acquisition of fetal cells (fetal nucleated red blood cells) is a significantly large task in addition to performing this safe prenatal diagnosis. In addition, it is known that there are also mother-derived nucleated red blood cells in blood of a pregnant mother. Therefore, separation of fetus-derived nucleated red blood cells (fetal nucleated red blood cells) from mother-derived nucleated red blood cells is also a task to be solved in addition to realizing the safe prenatal diagnosis.

A method for amplifying genomes of nucleated cells separated from maternal blood using a filter, identifying fetus-derived cells using polymorphism marker and a Y chromosomal marker, and detecting a chromosomal abnormality of fetus-derived cells through fluorescence in situ hybridization (FISH) method is disclosed in JP2004-533243A as a prenatal diagnostic method of a fetus focusing on nucleated red blood cells in maternal blood.

In addition, a method for amplifying paralogous genes of DNAs of a plurality of fetal cells, defining the amount of amplification product using a sequencer, and analyzing trisomy, which is a numerical abnormality, from the proportion of alleles is disclosed in JP2004-531271 A.

In addition, a method for detecting dizygotic twin fetuses is disclosed in JP2012-513217A. A fetal identifier and a paternal allele are identified from a mixed sample in which nucleated red blood cells are collected from maternal blood without discriminating whether the nucleated red blood cells are derived from a mother or a fetus.

**SUMMARY OF THE INVENTION**

Regarding monotocous and dizygotic twins, the prenatal diagnostic method of a fetus focusing on nucleated red blood cells in maternal blood is disclosed as described above. However, in the method of JP2004-533243A in which the prenatal diagnostic method of monotocous is disclosed, in a case of multiple fetuses, there is a problem in that it is impossible to determine how many cells may be analyzed. In the method of JP2004-531271A, there is a problem in that it is impossible to select fetal cells in maternal blood and to identify mother-derived cells and fetus-derived cells. Furthermore, in the method of JP2012-513217Ain which the prenatal diagnostic method of dizygotic twins is disclosed, detection is performed from a mixed sample of mother-derived cells and fetus-derived cells, and therefore, there is a problem in that it is impossible to grasp the origin of genetic information of individual twin fetuses.

In contrast, a method for detecting fetus-derived nucleic acids contained in a trace amount of maternal blood is performed as the noninvasive discrimination method of chromosomal heteroploidy of a fetus in the related art. However, in the conventional method, in a case where fetuses are twin fetuses, it is difficult to discriminate whether fetus-derived nucleic acids are derived from one of the twin fetuses or derived from both of the twin fetuses.

In addition, a method for detecting chromosomal heteroploidy after isolating fetus-derived nucleated red blood cells contained in maternal blood has also been known. However, in a case of twin fetuses, when it is impossible to grasp zygosity or sexuality thereof, the number of cells to be isolated is not clear, and there is a possibility of detecting fetus-derived nucleated red blood cells of only one fetus while missing the other fetus. Because of this, there is a problem such as poor efficiency of work since the number of cells required to be isolated is unclear.

An object of the present invention is to provide a noninvasive discrimination method of chromosomal heteroploidy of a fetus which can detect chromosomal heteroploidy of each fetus in a case where fetuses are monotocous or twin fetuses.

In addition, another object of the present invention is to provide a noninvasive discrimination system of chromosomal heteroploidy of a fetus which can detect chromosomal heteroploidy of each fetus in a case where fetuses are monotocous or twin fetuses.

The present inventors have conducted extensive studies in order to solve the above-described problems. As a result, they have found that, in the noninvasive discrimination method of chromosomal heteroploidy of a fetus which has a step of analyzing DNA of a candidate cell of a nucleated red blood cell isolated from maternal blood, it is possible to detect chromosomal heteroploidy of each fetus in a case where fetuses are monotocous or twin fetuses if fetuses are fetuses classified into any one case selected from monotocous, monochorionic monoamniotic twin fetuses, monochorionic diamniotic twin fetuses, and dichorionic diamniotic twin fetuses based on results of ultrasonic inspections and the number of candidate cells of nucleated red blood cells to be used for analyzing DNA is optimized based on the above-described classification, thereby completing the present invention.

In addition, the present inventors have found that, according to a noninvasive discrimination system of chromosomal heteroploidy of a fetus which includes fetus information acquisition means for acquiring classification information of fetuses based on results of ultrasonic inspections, form information acquisition means for acquiring form information of cells contained in a maternal blood sample, cell isolation means for isolating the cells contained in the maternal blood sample, a polymerase chain reaction (PCR) device of performing whole genome amplification on the isolated cells, a multiplex PCR device of performing multiplex PCR on the whole genome amplification products, and a DNA sequencing device of decoding a base sequence of the PCR amplification product and measuring the number of sequence leads, it is possible to detect chromosomal heteroploidy of each fetus in a case where fetuses are monotocous or twin fetuses, and have completed the present invention.

That is, the present invention provides the following (1) to (5).

(1) A noninvasive discrimination method of chromosomal heteroploidy of a fetus, the method comprising: a step of analyzing DNA of a candidate cell of a nucleated red blood cell isolated from maternal blood, in which the fetuses are fetuses classified into any one case selected from monotocous, monochorionic monoamniotic twin fetuses, monochorionic diamniotic twin fetuses, and dichorionic diamniotic twin fetuses based on results of ultrasonic inspections and the number of candidate cells of nucleated red blood cells to be used for analyzing the DNA is optimized based on the classification.

(2) The noninvasive discrimination method of chromosomal heteroploidy of a fetus according to (1), in which the optimization includes setting of the number of candidate cells of nucleated red blood cells used for analyzing the DNA as number of cases of monotocous $\leq$ number of cases of monochorionic monoamniotic twin fetuses < number of cases of monochorionic diamniotic twin fetuses < number of cases of dichorionic diamniotic twin fetuses.

(3) The noninvasive discrimination method of chromosomal heteroploidy of a fetus according to (1) or (2), in which the step of analyzing DNA of a candidate cell of a nucleated red blood cell isolated from maternal blood includes the following steps:

<step 1> a step of acquiring classification information of fetuses based on the membranous information obtained through an ultrasonic inspection, the classification information being shown in the following a) to d),

a) monotocous,
b) monochorionic monoamniotic twin fetuses,
c) monochorionic diamniotic twin fetuses, and
d) dichorionic diamniotic twin fetuses,

<step 2> a step of determining the number of candidate cells of nucleated red blood cells isolated from a maternal blood sample, based on the classification information,
<step 3> a step of isolating the above-described number of candidate cells of nucleated red blood cells from the maternal blood sample,
<step 4> a step of performing whole genome amplification on each of the isolated candidate cells of nucleated red blood cells,
<step 5> a step of performing genetic polymorphism analysis and/or a Y chromosome existence check, using whole genome amplification product,
<step 6> a step of comparing the number of genotypes of fetus-derived nucleated red blood cells which has been detected through the genetic polymorphism analysis and/or the Y chromosome existence check with the number of genotypes of fetuses which has been estimated from the classification information of the fetuses and selecting a process to proceed to the next step in a case where both numbers of genotypes are coincident with each other or a process to return to step 3 in a case where both numbers of genotypes are not coincident with each other, and
<step 7> a step of discriminating chromosomal heteroploidy of the fetus-derived nucleated red blood cells.

(4) A noninvasive discrimination system of chromosomal heteroploidy of a fetus, the system comprising: fetus information acquisition means for acquiring classification information of fetuses based on results of ultrasonic inspections;
form information acquisition means for acquiring form information of cells contained in a maternal blood sample, based on the classification information of fetuses; cell isolation means for isolating the cells contained in the maternal blood sample; a PCR device of performing whole genome amplification on the isolated cells; a multiplex PCR device of performing multiplex PCR on the whole genome amplification products; and a DNA sequencing device of decoding a base sequence of the PCR amplification product and measuring the number of sequence leads.
(5) The noninvasive discrimination system of chromosomal heteroploidy of a fetus according to (4), in which the number of candidate cells of nucleated red blood cells to be isolated is determined based on the information acquired by the fetus information acquisition means, the candidate cells of nucleated red blood cells to be isolated are selected based on the information acquired by the form information acquisition means, the candidate cells of nucleated red blood cells are isolated by the isolation means, whole genome amplification is performed on the candidate cells of nucleated red blood cells isolated using the PCR device, multiplex PCR is performed using the multiplex PCR device, and measurement of the number of sequence leads and decoding of base sequences of DNA fragments obtained through multiplex PCR using the DNA sequencing device are performed, the number of genotypes of candidate cells of nucleated red blood cells detected through genetic polymorphism analysis and/or a Y chromosome existence check with the number of genotypes of fetuses estimated from classification of fetuses, the process proceeds to the next step in a case where both numbers of genotypes are coincident with each other, and the process returns to the step of isolating the candidate cells of nucleated red blood cells in a case where both numbers of genotypes are not coincident with each other to repeat the steps thereafter.

According to the present invention, it is possible to provide the noninvasive discrimination method of chromosomal heteroploidy of a fetus in which it is possible to detect chromosomal heteroploidy of each fetus in a case where fetuses are monotocous or twin fetuses.

In addition, according to the present invention, it is possible to provide a noninvasive discrimination system of chromosomal heteroploidy of a fetus in which it is possible to detect chromosomal heteroploidy of each fetus in a case where fetuses are monotocous or twin fetuses.

In discriminating of chromosomal heteroploidy in the related art in which cell-free fetus-derived nucleic acids in plasma and serum of a mother are used, it is difficult to detect fetuses having chromosomal heteroploidy or the proportion of fetuses having chromosomal heteroploidy. However, according to the present invention, it is possible to determine zygosity of multiple fetuses using membranous information obtained through an ultrasonic inspection, determine the number of treatments of fetal nucleic acids associated with zygosity information, accurately discriminate chromosomal heteroploidy of individual fetus through genetic analysis of the determined number of treatments, and detect the proportion of fetuses having chromosomal heteroploidy.

## BRIEF DESCRIPTION OF THE DRAWINGS

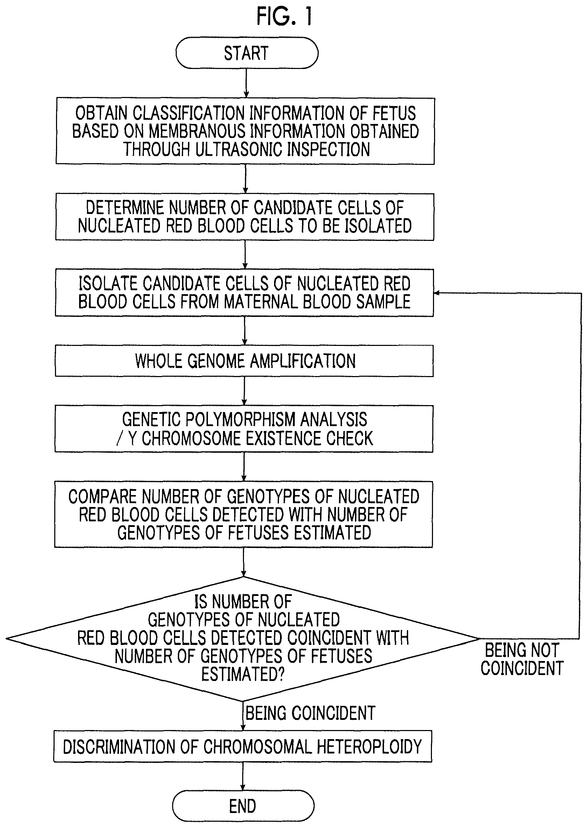

Fig. 1 is a flowchart showing an outline of a specific embodiment of the present invention.

## DESCRIPTION OF THE PREFERRED EMBODIMENTS

[Noninvasive Discrimination Method of Chromosomal Heteroploidy of Fetus]

The noninvasive discrimination method of a fetus of the present invention is [1] a noninvasive discrimination method of chromosomal heteroploidy of a fetus, the method including: a step of analyzing DNA of a candidate cell of a nucleated red blood cell isolated from maternal blood, in which the above-described fetuses are fetuses classified into any one case selected from monotocous, monochorionic monoamniotic twin fetuses, monochorionic diamniotic twin fetuses, and dichorionic diamniotic twin fetuses based on results of ultrasonic inspections and the number of candidate cells of nucleated red blood cells to be used for analyzing the above-described DNA is optimized based on the above-described classification.

More specifically, in the noninvasive discrimination method of a fetus of the present invention, it is preferable that [2] the above-described optimization includes setting of the number of candidate cells of nucleated red blood cells used for analyzing the above-described DNA as "number of cases of monotocous $\leq$ number of cases of monochorionic monoamniotic twin fetuses < number of cases of monochorionic diamniotic twin fetuses < number of cases of dichorionic diamniotic twin fetuses".

Still more specifically, in the noninvasive discrimination method of a fetus of the present invention, it is preferable that [3] the above-described step of analyzing DNA of a candidate cell of a nucleated red blood cell isolated from maternal blood includes the following steps:

<step 1> a step of acquiring classification information of fetuses is acquired based on the membranous information obtained through an ultrasonic inspection, the classification information being shown in the following a) to d)

a) monotocous,
b) monochorionic monoamniotic twin fetuses,
c) monochorionic diamniotic twin fetuses, and
d) dichorionic diamniotic twin fetuses,

<step 2> a step of determining the number of candidate cells of nucleated red blood cells isolated from a maternal blood sample, based on the above-described classification information,
<step 3> a step of isolating the above-described number of candidate cells of nucleated red blood cells from the maternal blood sample,
<step 4> a step of performing whole genome amplification on each of the isolated candidate cells of nucleated red blood cells,
<step 5> a step of performing genetic polymorphism analysis and/or a Y chromosome existence check, using whole genome amplification product,
<step 6> a step of comparing the number of genotypes of fetus-derived nucleated red blood cells detected through the above-described genetic polymorphism analysis and/or the above-described Y chromosome existence check with the number of genotypes of fetuses which is estimated from the classification information of the above-described fetuses and selecting a process to proceed to the next step in a case where both numbers of genotypes are coincident with each other or a process to return to step 3 in a case where both numbers of genotypes are not coincident with each other, and
<step 7> a step of discriminating chromosomal heteroploidy of the above-described fetus-derived nucleated red blood cells.

In the present invention, unless otherwise specified, "embryos" are also treated as "fetuses".
Hereinafter, the noninvasive discrimination method of chromosomal heteroploidy of a fetus of the present invention will be described in detail.

<Step 1 and Step 2>

(Classification Information of Fetus)

In the present invention, fetuses are classified into any one of the following four cases based on membranous information obtained through an ultrasonic inspection.

a) monotocous
b) monochorionic monoamniotic twin fetuses
c) monochorionic diamniotic twin fetuses
d) dichorionic diamniotic twin fetuses

The method for acquiring the classification information of fetuses is not particularly limited, but it is possible to acquire the classification information using well-known information transmission means and information reception means. Even if the classification information of fetuses is encoded, the classification information may be decoded. Even if the classification information of fetuses is encrypted, the classification information may be decrypted. Examples of the information transmission means include an electric telecommunication line, a magnetic disk, a telephone line, and a document, but are not limited thereto. Examples of the information reception means include a terminal device connected to an electric telecommunication line, a magnetic disk reader, a receiver, and document receiving means, but are not limited thereto.

(Ultrasonic inspection)

An ultrasonic inspection refers to an inspection method in which an ultrasonic wave is applied to an object and an echo thereof is imaged and which is intended for determination or diagnosis performed based on the image.

(Membranous Information)

Membranous information refers to the number of chorions and the number of amnions which have been diagnosed through an ultrasonic inspection, and a combination thereof. In the ultrasonic inspection, the diagnosis of membrane properties can be performed by directly counting the number of fetal sacs, the number of chorions, and the number of amnions. In addition, the diagnosis of membrane properties can also be performed in accordance with the thickness of the diaphragm on an ultrasonic image or the shape of a marginal region of the diaphragm. The period for performing diagnosis of membrane properties through the ultrasonic inspection is preferably an early pregnancy period. Specifically, before the 15th week of pregnancy is preferable and before the 10th week of pregnancy is more preferable. In addition, it is preferable to perform at least diagnosis whether fetuses are monochorionic twin fetuses or dichorionic twin fetuses. In many cases, as the period in which an ultrasonic inspection can be performed, the point in time when a pregnant woman becomes aware of pregnancy is generally the period in which an ultrasonic inspection can be performed. However, in general, it is possible to perform diagnosis after the 6th week to 8th week of pregnancy whether fetuses are at least monochorionic twin fetuses or dichorionic twin fetuses. In addition, a plurality of ultrasonic inspections may be performed.

(Types of Twin Fetuses)

The types of twin fetuses can be classified into three types of dichorionic diamniotic twin fetuses, monochorionic diamniotic twin fetuses, and monochorionic monoamniotic twin fetuses in accordance with the combination of the number of chorions and the number of amnions.

In dichorionic twin fetuses, it is possible to check two fetal sacs and an embryonic beat or a heartbeat of a fetus in each sac through an ultrasonic inspection. In monochorionic twin fetuses, it is possible to check, in general, one fetal sac between the 7th to 9th week of pregnancy and two embryonic beats or two heartbeats of fetuses in the sac through an ultrasonic inspection. In addition, there are two amnions in dichorionic twin fetuses. However, in monochorionic twin fetuses, there is a case where there are two amnions and a case where there is one amnion depending on the time period when a fertilized egg is divided into two.

In a case of monotocous, there is a chorion and an amnion.

(Relationship between Zygosity and Membrane Properties)

In addition, as the relationship between zygosity and membrane properties, in a case of dizygotic twin fetuses, the fetuses become dichorionic twin fetuses, and in a case of monozygotic twin fetuses, the fetuses become dichorionic twin fetuses or monochorionic twin fetuses depending on the time period when a fertilized egg is divided into two. There is a rare case where the fetuses become dizygotic twin fetuses, and at the same time, monochorionic twin fetuses. In addition, in the case of monochorionic twin fetuses, the fetuses are almost certainly monozygotic twin fetuses. In the case of dichorionic twin fetuses, there is a case where the fetuses are monozygotic twin fetuses and a case where the fetuses are dizygotic twin fetuses. There are few cases that fetuses are dizygotic even in the case of monochorionic twin fetuses. Furthermore, in a case where the genders of fetuses are different from each other, the fetuses are dizygotic twin fetuses without depending on membranous information, and in a case where the genders of fetuses are the same as each other, the fetuses are either of monozygotic twin fetuses or dizygotic twin fetuses. However, it is impossible to distinguish fetuses depending on only the genders of fetuses and the membranous information.

However, it is known that, in a case where fetuses are monozygotic twin fetuses, the proportion of monochorionic diamniotic twin fetuses is about 75%, the proportion of dichorionic diamniotic twin fetuses is about 25%, and the proportion of monochorionic monoamniotic twin fetuses is about 1%. For this reason, in a case where fetuses are diagnosed as monochorionic diamniotic twin fetuses through an ultrasonic inspection, the fetuses may be estimated as monozygotic twin fetuses, and in a case where fetuses are diagnosed as monochorionic diamniotic twin fetuses, the fetuses may be regarded as monozygotic twin fetuses. In a case where the genders of fetuses are different from each other, the fetuses may be treated as dizygotic twin fetuses without depending on membranous information.

(Relationship between Zygosity and Genotype)

In monozygotic twin fetuses, the two fetuses are derived from the same fertilized egg. Genotypes of the two fetuses are coincident with each other and the number of genotypes is one. In contrast, in dizygotic twin fetuses, the two fetuses are respectively derived from fertilized eggs different from each other. Therefore, the genotypes of the two fetuses are not coincident with each other and the number of genotypes is two.

(Number of Candidate Cells of Nucleated Red Blood Cells to be Used for Analyzing DNA Isolated from Maternal Blood Sample)

The number of candidate cells of nucleated red blood cells isolated from a maternal blood sample is determined based on classification information of a fetus.

The number of genotypes detected through genotype analysis (in the present invention, "genetic polymorphism analysis" and "Y chromosome existence check" are collectively called) is estimated as two in dichorionic diamniotic twin fetuses, and as one in monochorionic diamniotic twin fetuses, monochorionic monoamniotic twin fetuses, and monotocous, from the above-described relationship between membrane properties and zygosity and the above-described relationship between zygosity and genotypes. However, even in the case of dichorionic diamniotic twin fetuses, there is a possibility that the fetuses may be monozygotic twin fetuses, and even in the case of monochorionic diamniotic twin fetuses or monochorionic monoamniotic twin fetuses, there is little possibility that the fetuses may be dizygotic twin fetuses. The possibility is larger in monochorionic diamniotic twin fetuses than in monochorionic monoamniotic twin fetuses, and the possibility in monochorionic monoamniotic twin fetuses is the same as or slightly larger than the possibility in monotocous. For this reason, the relationship between large number or small number of candidate cells of nucleated red blood cells to be isolated is as shown in the following inequation.

Number of cases of monotocous $\leq$ number of cases of monochorionic monoamniotic twin fetuses < number of cases of monochorionic diamniotic twin fetuses < number of cases of dichorionic diamniotic twin fetuses.

[Step 3]

(Maternal Blood Sample)

In the present invention, maternal blood is peripheral blood of a pregnant woman, on which an ultrasonic inspection is performed or is to be performed. A maternal blood sample is peripheral blood collected from a pregnant woman or blood obtained by adding an anticoagulant to peripheral blood collected from a pregnant woman and performing a treatment such as dilution with a physiological salt solution. A pregnant woman for whom membranous diagnosis is performed by performing an ultrasonic inspection and a pregnant woman from whom maternal blood is collected are the same individuals.

Mother-derived leucocytes such as eosinophils, neutrophils, basophils, monocytes, and lymphocytes; mature red blood cells having no nucleus; mother-derived nucleated red blood cells; and fetus-derived nucleated red blood cells are included in maternal blood. It is known that fetus-derived nucleated red blood cells exist in maternal blood after about the 6th week of pregnancy. For this reason, in the present invention, maternal blood of a pregnant woman which has been collected after about the 6th week of pregnancy is tested.

Candidate cells of nucleated red blood cells are isolated from a maternal blood sample after determining the number of candidate cells of nucleated red blood cells to be isolated, after obtaining results of classifying fetuses based on results of an ultrasonic inspection. Therefore, in a case of performing membranous diagnosis using the ultrasonic inspection later than blood collection, it is preferable to refrigerate the maternal blood sample at least until the membranous diagnosis is performed.

Fetus-derived nucleated red blood cells are erythrocyte precursors which exist in maternal blood after passing through the placenta. Red blood cells of a fetus can be nucleated during pregnancy of a mother. Since there are chromosomes in these red blood cells, it is possible to obtain fetus-derived chromosomes and fetal genes through less invasive means. It is known that these fetus-derived nucleated red blood cells exist at a ratio of one fetus-derived nucleated red blood cell to about $10^6$ cells in maternal blood. Therefore, the existence probability of the fetus-derived nucleated red blood cells in peripheral blood of a pregnant woman is significantly low.

The density of blood cells of a mother containing nucleated red blood cells of a fetus is disclosed in WO2012/023298A. According to the disclosure, the density of the fetus-derived nucleated red blood cells which is assumed is about 1.065 to 1.095 g/mL, the density of blood cells of a mother is about 1.070 to 1.120 g/mL in a case of red blood cells, about 1.090 to 1.110 g/mL in a case of eosinophils, about 1.075 to 1.100 g/mL in a case of neutrophils, about 1.070 to 1.080 g/mL in a case of basophils, about 1.060 to 1.080 g/mL in a case of lymphocytes, and about 1.060 to 1.070 g/mL in a case of monocytes.

(Concentration of Nucleated Red Blood Cell)

In order to isolate nucleated red blood cells from a maternal blood sample, it is desirable to first concentrate the nucleated red blood cells in the maternal blood sample through a discontinuous density gradient centrifugation method.

The discontinuous density gradient centrifugation method is a method for forming discontinuous density gradient using a first medium and a second medium which have different densities from each other and fractionating and concentrating components having an intermediate density therebetween.

As the first medium and the second medium used in the present invention, it is possible to use media such as PERCOLL (registered trademark, Percoll, manufactured by Sigma-Aldrich Co. LLC.) which is a silica colloid particle dispersion liquid which has a diameter of 15 to 30 nm and is coated with polyvinyl pyrrolidone, FICOLL-PAQUE (registered trademark, Ficoll-Paque, manufactured by GE Healthcare Life Sciences) which is a neutral hydrophilic polymer rich in a side chain made of sucrose, and HISTOPAQUE (registered trademark, Histopaque, manufactured by Sigma-Aldrich Co. LLC.) which is a solution using polysucrose and sodium diatrizoate. In the present invention, it is preferable to use PERCOLL and/or HISTOPAQUE. A product of PERCOLL having a density (gravity) of 1.130 is commercially available and it is possible to prepare a medium having a target density (gravity) through dilution. It is possible to prepare the first medium and the second medium having a desired density using a medium having a density of 1.077 g/mL and a medium having a density of 1.119 g/mL which are commercially available as HISTOPAQUE.

The density of media to be stacked is set in order to separate nucleated red blood cells having a density of about 1.065 to 1.095 g/mL from other blood cells in a maternal blood sample. The central value of a density distribution of nucleated red blood cells is about 1.080 g/mL. Therefore, it is possible to collect fractions containing nucleated red blood cells on an interface between two adjacent media having different densities interposing the density by making the media overlap each other. It is preferable that the density of the first medium is 1.08 g/mL to 1.10 g/mL and the density of the second medium is greater than or equal to 1.06 g/mL and less than 1.08 g/mL. It is more preferable that the density of the first medium is 1.08 g/mL to 1.09 g/mL and the density of the second medium is greater than or equal to 1.065 g/mL and less than 1.08 g/mL. For example, it is possible to separate plasma components, eosinophils, and monocytes from fractions containing nucleated red blood cells, by setting the density of the first medium to 1.085 g/mL and the density of the second medium to 1.075 g/mL. In addition, it is also possible to partially separate red blood cells, neutrophils, and lymphocytes therefrom. In the present invention, the type of the first medium and the type of the second medium is not limited as long as it is possible to realize the effect of the present invention, and may be the same as or different from each other. However, it is preferable to use the same types of media.

(Acquisition of Form Information of Cell)

In the present invention, a specimen for analysis is produced by coating the top of a transparent substrate, preferably a slide glass with fractions containing nucleated red blood cells separated from a maternal blood sample. Candidate cells of fetus-derived nucleated red blood cells are sorted out using the form information of cells using this specimen. In the present invention, it is possible to sort out candidate cells of fetus-derived nucleated red blood cells using the ratio of the area of a nuclear region to the area of cytoplasm of a cell, the degree of circularity of a nucleus, the area of a nuclear region, and the like. However, it is preferable to sort out cells having conditions, which satisfy the ratio of the area of a nuclear region to the area of cytoplasm or the degree of circularity of a nucleus, as candidate cells of fetus-derived nucleated red blood cells.

In the present invention, it is preferable to sort out candidate cells of nucleated red blood cells satisfying the following Formula (I).

$$0.25 < N / C < 1.0 \qquad \cdots (I)$$

However, in Formula (I),

C represents the area ($\mu m^2$) of cytoplasm, and
N represents the area ($\mu m^2$) of a nuclear region.

In addition, in the present invention, it is more preferable to sort out candidate cells of nucleated red blood cells satisfying the following Formula (II).

$$0.65 < N / L^2 < 0.785 \qquad \cdots (II)$$

However, in Formula (II),

L represents the length of the major axis of a nucleus of a cell to be subjected to image analysis, and
N has the same meaning as N in the above-described Formula (I).

The length of the monoamniotic of a nucleus of a cell to be subjected to image analysis is a length of the major axis circumscribing a cell nucleus which has a complicated shape.

In the present invention, a system of sorting out candidate cells of fetus-derived nucleated red blood cells using form information of cells is equipped with an optical microscope, a digital camera, a stage for slide glass, an optical transfer system, an image processing PC, a control PC, and a display. The optical transfer system includes an objective lens and a CCD camera. The image processing PC includes a processing system of performing data analysis and storing of data. The control PC includes a control system of controlling the position of a stage for slide glass or controlling the entire processing.

<Step 4>

(Whole Genome Amplification)

Whole genome amplification is an amplification method in which it is possible to amplify a trace amount of DNA collected from a single cell to, for example, about $10^6$ times and it is easy to perform genetic analysis such as genetic mutation using the amplification product.

Regarding each candidate cell of a nucleated red blood cell isolated from a maternal blood sample, genome DNA obtained by being separated from a cell through cytolysis, which is a usual method and in which a surfactant is used, and a protein decomposition step in which protease K or the like is used for the whole genome amplification method used in the present invention.

In addition, a cell may be added to a PCR reaction solution as it is without separating genome DNA from the cell.

It is possible to use a reagent PicoPLEX WGA kit (manufactured by New England Biolabs) based on a polymerase chain reaction (PCR), a GenomePlex Single Cell Whole Genome Amplification kit (manufactured by Sigma-Aldrich Co. LLC.), and a MALBAC method (WO2012/166425A) as a whole genome amplification reagent. In addition, it is possible to similarly use a reagent GenomiPhi (manufactured by GE Healthcare) and REPLI-g (manufactured by QIAGEN) based on a chain-substituting DNA synthesis reaction. In the present invention, it is preferable to use a PicoPLEX WGA kit (manufactured by New England Biolabs).

It is preferable to check whether or not amplification is performed on an amplification product (whole genome amplification product) which has been obtained through whole genome amplification, through agarose gel cataphoresis. Furthermore, it is preferable to refine the whole genome amplification product using QIAquick PCR Purification Kit (manufactured by QIAGEN).

In addition, the concentration of a whole genome amplification product is preferably measured using a device, such as NanoDrop (manufactured by Thermo Fisher Scientific Inc.), Bioanalyzer (manufactured by Agilent Technologies), or Quantus Fluorometer (manufactured by Promega KK.) which can measure the concentration of DNA.

<Step 5 and Step 6>

«Genetic Polymorphism Analysis»

Although candidate cells of nucleated red blood cells, which have been subjected to whole genome amplification, are obtained by performing whole genome amplification on candidate cells of fetus-derived nucleated red blood cells which have been sorted out and isolated based on form information, a case where fetus-derived nucleated red blood cells are not contained or a case where mother-derived nucleated red blood cells are mixed in can be considered.

For this reason, it is preferable to identify fetus-derived nucleated red blood cells from the candidate cells of nucleated red blood cells which have been subjected to whole genome amplification, through genetic polymorphism analysis and/or the Y chromosome existence check, as an embodiment.

In the genetic polymorphism analysis, genetic polymorphism such as short tandem repeat (STR) which is a short repetitive sequence, SNPs which is single base polymorphism, or the like is identified. In the present invention, identification of genetic polymorphism of candidate cells of nucleated red blood cells after performing PCR amplification by targeting a region including a genetic polymorphism site, and decoding a base sequence of the obtained PCR product is a preferred aspect. In the PCR amplification, a plurality of times of PCR amplification in which individual regions are targeted may be performed. However, multiplex PCR is preferable from the viewpoint of improving throughput.

In order to perform genetic polymorphism analysis, the region on which PCR amplification is to be performed is preferably set as a region which includes a genetic polymorphism site such as a STR site or an SNPs site and includes a chromosome-specific base sequence, and on which it is possible to chromosome-specifically perform PCR amplification. The PCR amplification product obtained through the PCR amplification can be used not only for performing genetic polymorphism analysis but also for performing discrimination of chromosomal heteroploidy. Therefore, the PCR amplification product is efficient. Furthermore, it is also possible to improve throughput while performing the genetic polymorphism analysis and the detection of chromosomal heteroploidy at the same time.

Regarding candidate cells of nucleated red blood cells, it is possible to discriminate nucleated red blood cells, which have genetic polymorphism information different from that of a mother by comparing genetic polymorphism information of the mother with genetic polymorphism information obtained through genetic polymorphism analysis, as fetus-derived nucleated red blood cells. The genetic polymorphism information of the mother may be genetic polymorphism information obtained through genetic polymorphism analysis in advance, or may be a genotype determined by performing the same genetic polymorphism analysis on mother-derived cells, for example, white blood cells, which have been isolated from maternal blood.

Regarding target regions of multiplex PCR in the present invention, it is intended to detect heteroploidy of mainly chromosome 13, chromosome 18, or chromosome 21 in fetus-derived nucleated red blood cells each in twin fetuses. Therefore, it is preferable to select at least 10 regions in total which are specific to each of these chromosomes and it is more preferable to select at least 15 regions. Furthermore, these regions are preferably regions including a genetic polymorphism site. Regarding the size of a target region, it is preferable to design a primer pair excluding a primer portion such that greater than or equal to 40 bp of the region is amplified, and 40 bp to 200 bp is more preferable.

(PCR)

In a polymerase chain reaction (PCR), template DNA is repeatedly replicated using DNA polymerase. Replication is started using polymerase after adding a short DNA primer hybridizing to the template DNA in a starting portion and an ending portion of a DNA base sequence to be amplified. Two chains of template double-stranded DNA are dissociated and are individually replicated every time the replication is repeated.

(Multiplex PCR)

In multiplex PCR, it is possible to use heat-resistant DNA polymerase and a reaction buffer which are generally used in PCR. However, in some cases, each primer pair has a different temperature annealing to template DNA, and therefore, it is necessary to examine reaction conditions. For this reason, it is preferable to use heat-resistant DNA polymerase and reaction buffer which are optimized for multiplex PCR. In the present invention, it is more preferable to cause a reaction using Multiplex PCR Assay Kit (manufactured by TAKARA BIO INC.).

(Analysis of Sequence of Amplification Product)

In a product amplified through multiplex PCR, it is preferable to check the presence or absence of amplification through agarose gel cataphoresis. Furthermore, it is preferable to refine the multiplex PCR amplification product using QIAquick PCR Purification Kit (manufactured by QIAGEN). In addition, it is also possible to use AMPure XP Kit (manufactured by BECKMAN COULTER) which is a purification method in which paramagnetic microbeads are used.

In addition, it is preferable that the concentration of the multiplex PCR amplification product is measured using a device, such as NanoDrop (manufactured by Thermo Fisher Scientific Inc.), Bioanalyzer (manufactured by Agilent Technologies), or Quantus Fluorometer (manufactured by Promega KK.) which can measure the concentration of DNA.

It is possible to use Miseq (manufactured by Illumina, Inc.) for analyzing a sequence of an amplification product. In a case of measuring a plurality of multiplex PCR amplification products using Miseq, it is necessary to add P5 and P7 sequences, which are used for hybridizing to a sample identification sequence (index sequence) formed of 6 to 8 bases, and an oligonucleotide sequence on the top of a Miseq flow cell, to each of the multiplex PCR amplification products. By adding these sequences thereto, it is possible to measure up to 96 types of multiplex PCR amplification products at a time.

It is possible to use an adapter ligation method or a PCR method as the method for adding an index sequence and P5 and P7 sequences to both terminals of the multiplex PCR amplification products.

In addition, in a case of mixing a plurality of multiplex PCR amplification products and measuring the plurality of multiplex PCR amplification products using Miseq, it is desirable to accurately quantitatively determine each PCR product. It is possible to use Bioanalyzer (manufactured by Agilent Technologies), or Quantus Fluorometer (manufactured by Promega KK.). However, a method for measuring the multiplex PCR amplification products through a quantitative PCR method. It is preferable to perform quantitative determination as the quantitative method using KAPA Library Quantification Kits manufactured by NIPPON Genetics Co, Ltd.

As the method for analyzing sequence data obtained using Miseq, it is preferable to map the sequence data in a well-known human genome sequence using Burrows-Wheeler Aligner (BWA). As means for analyzing a genetic abnormality, it is preferable to analyze genetic mutation or quantitative determination of the number of chromosomes, using SAMtools and BEDtools.

«Y Chromosome Existence Check»

There is no Y chromosome in mother-derived nucleated red blood cells, and therefore, detection of a Y chromosome is strong evidence indicating that the cells are derived from a fetus.

Examples of the method for checking the existence of a Y chromosome include a method for checking whether a correct amplification product is generated after performing a PCR reaction by targeting a region on a Y chromosome which includes a Y chromosome-specific base sequence and on which PCR amplification can be performed specifically to the Y chromosome. It is possible to check whether a correct amplification product is generated, by checking whether a band having a desired size can be obtained through agarose gel cataphoresis, or by checking whether a predetermined region is amplified after decoding a base sequence of a PCR amplification product.

If a region including a genetic polymorphism site is selected as a target region, it is possible to use the region also for analysis of genetic polymorphism, and therefore in some cases, it is useful in a case where both of the genders of twin fetuses are male.

Examples of another method for checking the existence of a Y chromosome include a FISH method in which a Y chromosome-specific fluorescent prove is used. Specific examples of the method in which CEP X/Y DNA Probe Kit manufactured by Abbott is used.

«Comparison of Number of Genotypes»

The number of genotypes of fetuses which is estimated from classification information is one in cases of a monotocous, monochorionic monoamniotic twin fetuses, and monochorionic diamniotic twin fetuses, and two in a case of dichorionic diamniotic twin fetuses.

The number of genotypes of nucleated red blood cells detected through genetic analysis (in the present invention, "genetic polymorphism analysis" and "Y chromosome existence check" are collectively called) is greater than or equal to 0, and the number of genotypes of fetuses, which is estimated from classification information, plus 1 is set as an upper limit.

In a case where the number of genotypes of fetus-derived nucleated red blood cells which has been detected through genetic analysis is not coincident with (does not match) the number of genotypes of fetuses which is estimated from classification information, the process returns to step 3 to repeat the steps after step 3. The number of times of returning from step 7 to step 3 is not particularly limited, but is preferably once to five times and more preferably once to three times.

In a case where the number of genotypes of fetus-derived nucleated red blood cells detected through genetic analysis is coincident with the number of genotypes of fetuses estimated from classification information, the process proceeds to step 7 to discriminate chromosomal heteroploidy of fetus-derived nucleated red blood cells.

In a case where the number of genotypes of nucleated red blood cells (including fetus-derived nucleated red blood cells and mother-derived nucleated red blood cells) detected through genetic analysis is the number of genotypes of fetuses, which is estimated from classification information, plus 1, the number of genotypes of fetus-derived nucleated red blood cells and the number of genotypes of fetuses are coincident with each other.

For example, it is possible to determine that, in a case where the detected number of genotypes regarding nucleated red blood cells when fetuses are classified in a case of any of monotocous, monochorionic monoamniotic twin fetuses, and monochorionic diamniotic twin fetuses is two, one type among the two types is a genotype of fetus-derived nucleated red blood cells, and in a case where the detected number of genotypes regarding nucleated red blood cells when fetuses are classified in a case of dichorionic diamniotic twin fetuses is three, two types among the three types are genotypes of fetus-derived nucleated red blood cells. In these cases, it is possible to discriminate chromosomal heteroploidy of fetus-derived nucleated red blood cells if discrimination of chromosomal heteroploidy is performed on all of the obtained two or three types of the genotypes even without comparing a genotype of a nucleated red blood cell with a genotype of a mother. Since there is no chromosomal heteroploidy in mother-derived nucleated red blood cells, detection of chromosomal heteroploidy shows that there is chromosomal heteroploidy in fetus-derived nucleated red blood cells.

In a case where the process returns to step 3 from step 7 to repeat the steps after step 3, when the number of genotypes of fetus-derived nucleated red blood cells which has been detected through genetic analysis is not coincident with the number of genotypes of fetuses, which is estimated from classification information, in step 7 again, information whether the genders of fetuses are the same as or different from each other is obtained. Then, in a case where the genders thereof are the same as each other, the fetuses are regarded as being monozygotic and it is desirable not to repeat the steps any more. In a case where the genders thereof are different from each other, the fetuses are regarded as being dizygotic, and therefore, it is desirable to repeat the steps several times, preferably 1 to 5 times, and more preferably 1 to 3 times.

<Step 7>

(Measurement of Number of Sequence Leads of Amplification DNA Fragment)

Regarding a nucleic acid fragment which has been amplified through polymerase chain reaction and in which fetal nucleated red blood cells are identified, the amplification amount of amplification product having a sequence of a region of 40 bp to 200 bp which has been previously determined is obtained using a sequencer. Regarding a cell which has been identified as a mother-derived nucleated red blood cell, the amplification amount of amplification product having a sequence of a region of 40 bp to 200 bp is analyzed as a standard (or a reference) using the sequencer. In a case where the fetuses are in a normal state, it is expected that the ratio of the amplification amount of mother-derived amplification product to the amplification amount of fetus-derived amplification product becomes almost 1:1. In a case where fetuses have a disease which is trisomy derived from an amplified chromosome, it is expected that the ratio thereof becomes almost 1:1.5 (or 2:3).

In the present invention, the proportions of the amount of fetus-derived amplification products to the amount of amplification products which have been collected from a plurality of pregnant mothers and derived from mothers who are pregnant with normal fetuses were obtained plural times, and the distribution thereof is obtained. In addition, the proportions of the amount of fetus-derived amplification products to the amount of amplification products derived from mothers who are pregnant with fetuses with trisomy, and the distribution thereof is obtained. It is also possible to set a cutoff value in a region where these two distributions do not overlap. After comparing the cutoff value which has been previously determined with a result in which the proportion of the amplification products is obtained, it is also possible to interpret inspection results that the fetuses are normal in a case where the proportion thereof is less than or equal to the cutoff value, and the fetuses have trisomy in a case where the proportion thereof is greater than or equal to the cutoff value.

[Noninvasive Discrimination System of Chromosomal Heteroploidy of Fetus]

The noninvasive discrimination system of chromosomal heteroploidy of a fetus of the present invention includes [1']: fetus information acquisition means for acquiring classification information of fetuses based on results of ultrasonic inspections; form information acquisition means for acquiring form information of cells contained in a maternal blood sample, based on the classification information of fetuses; cell isolation means for isolating the cells contained in the maternal blood sample, a PCR device of performing whole genome amplification on the isolated cells; a multiplex PCR device of performing multiplex PCR on the whole genome amplification products; and a DNA sequencing device of decoding a base sequence of the PCR amplification product and measuring the number of sequence leads.

More specifically, in the noninvasive discrimination system of chromosomal heteroploidy of a fetus of the present invention, [2'] the number of candidate cells of nucleated red blood cells to be isolated is determined based on the information acquired by the above-described fetus information acquisition means, the candidate cells of nucleated red blood cells to be isolated are selected based on the information acquired by the above-described form information acquisition means, the candidate cells of nucleated red blood cells are isolated by the above-described isolation means, whole genome amplification is performed on the candidate cells of nucleated red blood cells isolated using the above-described PCR device, multiplex PCR is performed using the above-described multiplex PCR device, and measurement of the number of sequence leads and decoding of base sequences of DNA fragments obtained through multiplex PCR using the above-described DNA sequencing device are performed, the number of genotypes of candidate cells of nucleated red blood cells detected through genetic polymorphism analysis and/or a Y chromosome existence check with the number of genotypes of fetuses estimated from classification of fetuses, the process proceeds to the next step in a case where both numbers of genotypes are coincident with each other, and the process returns to the step of isolating the candidate cells of nucleated red blood cells in a case where both numbers of genotypes are not coincident with each other to repeat the steps thereafter.

Examples

[Example 1]

(Ultrasonic inspection)

A pregnant woman before the 10th week of pregnancy was tested using an ultrasonic inspection device, and a peripheral blood sample was obtained from a subject whose pregnancy of twin fetuses was confirmed. It is possible to use Vscan manufactured by GE Healthcare Life Sciences and an Aplio series manufactured by Toshiba Medical Systems Corporation as the ultrasonic inspection device. However, in the present invention, it is preferable to use FC1, FAZONE CB, and FAZONE M manufactured by Fujifilm Corporation.

(Acquisition of Classification Information of Fetus)

It was confirmed that membrane properties of the subject was dichorionic diamniotic twin fetuses, through the ultrasonic inspection. Both of the genders of the fetuses were male fetuses, and it was difficult to discriminate whether the fetuses are monozygotic twin fetuses or dizygotic twin fetuses. However, it was determined that there was a high possibility that the dichorionic diamniotic twin fetuses were stochastically dizygotic twin fetuses. Therefore, collecting 20 or more cells was aimed in a step of collecting a candidate cell of a nucleated red blood cell to be described below.

(Acquisition of Maternal Blood Sample)

10.5 mg of sodium salts of EDTA was added to a 7 mL blood collecting tube as an anticoagulant, and then, 7 mL of peripheral blood was obtained within the blood collecting tube as volunteer blood after obtaining informed consent from a pregnant woman volunteer on which the above-described ultrasonic inspection was performed. Thereafter, the blood was diluted using physiological salt solution.

(Concentration Step of Nucleated Red Blood Cell)

A liquid with a density of 1.070 g/mL and a liquid with a density of 1.095 g/mL were prepared using a Percoll liquid (registered trademark, Sigma-Aldrich Co. LLC.), 2 mL of a liquid with a density of 1.095 g/mL was added to the bottom portion of a centrifuge tube, and the centrifuge tube was cooled in a refrigerator for 30 minutes at 4°C. Thereafter, the centrifuge tube was taken out from the refrigerator and 2 mL of a liquid with a density of 1.070 g/mL was made to slowly overlap the top of the liquid with a density of 1.095 g/mL so as not to disturb an interface. Then, 11 mL of diluent of blood which had been collected in the above was slowly added to the top of the medium with a density of 1.070 g/mL in the centrifuge tube. Thereafter, centrifugation was performed for 20 minutes at 2000 rpm.

(Coating of Slide Glass)

The centrifuge tube was taken out and fractions which had been deposited between the liquid with a density of 1.070 g/mL and the liquid with a density of 1.095 g/mL were collected using a pipette. A slide glass substrate (hereinafter, referred to as a "slide glass substrate 1") was held by one hand and a droplet of the collected blood was spotted at one end of the slide glass substrate 1. A slide glass substrate (hereinafter, referred to as a "slide glass substrate 2") was held by the other hand and one end of the slide glass substrate 2 was brought into contact with the slide glass substrate 1 at 30°. The lower contact surface of the slide glass substrate 2 which was brought into contact with the fractions of blood was then spread into a space surrounded by the two slide glass substrates due to a capillary phenomenon. Next, the slide glass substrate 2 was made to be slid in a direction of a region opposite to the region of the slide glass substrate 1, on which blood was placed, while maintaining the angle, and the slide glass substrate 1 was uniformly coated with blood. After the completion of the coating, the slide glass substrate 1 was sufficiently dried through air blowing for one or more hours. This slide glass substrate was immersed in a MAY-Grunwald dyeing liquid for three minutes and was washed by being immersed in a phosphoric acid buffer solution. Thereafter, the slide glass substrate was immersed in a GIEMSA dyeing liquid (which was diluted with a phosphoric acid buffer solution to make a concentration of 3%) for 10 minutes. Thereafter, A plurality of dyed slide glass substrates were produced by being dried after being washed with pure water.

(Step of Sorting Out Candidate Cell of Fetus-Derived Nucleated Red Blood Cell Using Form information of Cell)

In order to sort out candidate cells of fetus-derived nucleated red blood cells from the cells with which the top of the slide glass substrate was coated, an optical microscope provided with an electric XY stage, an objective lens, and a CCD camera, a control unit provided with an XY stage control unit and a Z-direction control unit, and a control unit portion including an image input unit, an image processing unit, and an XY position recording unit were provided.

Blood cells which had been prepared as described above and with which the top of the slide glass substrate was coated were placed on the XY stage and scanning was performed by being focused on the slide glass. An image which had been obtained using an optical microscope was taken and nucleated red blood cells which were target cells were searched through image analysis.

In the image analysis, cells which satisfied the two following conditions were detected and the XY position was recorded.

$$0.25 < N / C < 1.0 \qquad \cdots (I)$$

$$0.65 < N /L^2 < 0.785 \qquad \cdots (II)$$

However, in Formulas (I) and (II),

C represents the area of cytoplasm of a cell on which image analysis is performed,
N represents the area of a nucleus of a cell on which image analysis is performed, and
L represents a length of a major axis of a nucleus of a cell on which image analysis is performed, that is a length of a major axis of an elliptical shape circumscribing a cell nucleus which has a complicated shape.

20 cells which satisfy Formulas (I) and (II) were selected from cells existing on the slide glass substrate as candidate cells of fetus-derived nucleated red blood cells.

(Isolation of Candidate Cell of Fetus-Derived Nucleated Red Blood Cell)

The 20 candidate cells of the fetus-derived nucleated red blood cells selected as described above were collected using a micromanipulator.

(Whole Genome Amplification)

Whole genome amplification was performed on each of the 20 cells which had been isolated using the micromanipulator, using a PicoPLEX WGA kit manufactured by New England Biolabs to amplify a trace amount of DNA in the cells into about a million times in accordance with description of a manual.
Impurities of the obtained whole genome amplification product were removed using QIAquick PCR Purification Kit (manufactured by QIAGEN), and the concentration of the amplification product was measured using Quantus Fluorometer dsDNA System (Promega KK.).

(Multiplex PCR Step)

Primers to be used for multiplex PCR were produced from 23 chromosome positions shown in Tables 1 and 2 for the purpose of analyzing fetal cells and analyzing a numerical abnormality of chromosomes. The primers were produced such that the PCR amplification base length of each detection region became 100 to 220 base pairs, and the positions of the primers were designed such that the inside of each detection region contained genetic polymorphism. The designed 46 types of primers were mixed with each other such that the final concentration of each primer became 25 nmol/L.
In multiplex PCR, the reaction was performed using a Multiplex PCR Assay kit (TAKARA BIO INC.).
A reaction solution of which the final amount was 25 $\mu$L and which contained 10 ng of an amplification product obtained from each cell as a template, 8 $\mu$L of a mixture of 46 primers, 0.125 $\mu$L of Multiplex PCR Mix 1, 12.5 $\mu$L of Multiplex PCR Mix 2, and water was prepared.
In the reaction, after modification performed for 60 seconds at 94°C, a cycle of 30 seconds at 94°C, 90 seconds at 60°C, and 30 seconds at 72°C was performed 30 times as PCR conditions.
The obtained PCR product was purified using QIAquick PCR Purification Kit (manufactured by QIAGEN).

[Table 1]

| SEQ ID No. | Primer name | Base sequences (5' → 3') | Gene name | Chromosome | SNP ID |
|---|---|---|---|---|---|
| 1 | rs2737699-F | CGCTCTTCCGATCTCTGTGCAGTCATCTTGCTCTACA | SACS | 13 | rs2737699 |
| 2 | rs2737699-R | CGCTCTTCCGATCTGACCAGTTTTGATGGCTCCGAAG | | | |
| 3 | rs9552929-F | CGCTCCGATCTCTGCCGCACAACAGGTAAAAGAC | SACS | 13 | rs9552929 |
| 4 | rs9552929-R | CGCTCTTCCGATCTGACACGGTATTTCCCTGGTTCTG | | | |
| 5 | rs3751355-F | CGCTCTTCCGATCTCTGGGGAATAAGGGTGACGTGGG | C1QTKF9 | 13 | rs3751355 |
| 6 | rs3751355-R | CGCTCTTCCGATCTGACCCAGCCTCTTATTCCCCGGA | | | |
| 7 | rs3751357-F | CGCTCTTCCGATCTCTGTGCTTTCACTCTGGGGCTCA | C1QTNF9 | 13 | rs3751357 |
| 8 | rs3751357-R | CGCTCTTCCGATCTGACATAGACCCCACCAATGTGGC | | | |
| 9 | rs202198487-F | CGCTCTTCCGATCTCTGGGCAACATTGGGCCTTTGGG | C1QTNF9 | 13 | rs202198487 |
| 10 | rs202198487-R | CGCTCTTCCGATCTGACCCTCGATCTCCTTTCCAGCC | | | |
| 11 | rs2230162-P | CGCTCTTCCGATCTCTGTGTCCATGCAAACCCTGGAA | MYOM1 | 18 | rs2230162 |
| 12 | rs2230162-R | CGCTCTTCCGATCTGACAAGTGGGGAGAGTGGCTGTT | | | |
| 13 | rs3745051-F | CGCTCTTCCGATCTCTGGCCATTGAGAAAGGCTGCTC | DLGAP1 | 18 | rs3745051 |
| 14 | rs3745051-R | CGCTCTTCCGATCTGACCAACAGTGCACAGTCTTGGG | | | |
| 15 | rs581894-F | CGCTCTTCCGATCTCTGCATCTCCAGGCTGTGCAGTG | SOGA2 | 18 | rs581894 |
| 16 | rs581894-R | CGCTCTTCCGATCTGACGGTCCCTTCCCACAAGCAG | | | |
| 17 | rs8096037-F | CGCTCTTCCGATCTCTGGCAGCAGGAGGAASGCAAAA | PIEZO2 | 18 | rs8096037 |
| 18 | rs8096037-R | CGCTCTTCCGATCTGACTCCCAAGCCTGACCATACCT | | | |
| 19 | rs7234309-F | CGCTCTCCGATCTCTGGCAACATGACAAGCACTGGG | PIEZO2 | 18 | rs7234309 |
| 20 | rs7234309-R | CGCTCTTCCGATCTGACCGCTACTGGGACTGGCTGAT | | | |
| 21 | rs9305426-F | CGCTCTTCCGATCTCTGCTGTGGTTATGGCGGCTGTG | KRTAP6-3 | 21 | rs9305426 |
| 22 | rs9305426-R | CGCTCTTCCGATCTGACAGGAGTGGGAGACATAGCCA | | | |
| 23 | rs12053674-F | CCCTCTTCCGATCTCTGAGCCACAGCCAGTTCCATAG | KRTAP21-2 | 21 | rs12053674 |
| 24 | rs12053674-R | CGCTCTTCCGATCTGACCTCCTGAAATCATGTGTTGC | | | |

[Table 2]

| SEQ ID No. | Primer name | Base sequence (5' → 3') | Gene name | Chromosome | SNP ID |
|---|---|---|---|---|---|
| 25 | rs10775648-F | CGCTCTTCCGATCTCTGCTTCGAGTCTGTGGATCGCG | HUNK | 21 | rs10775648 |
| 26 | rs10775648-R | CGCTCTTCCGATCTGACGCAAAGGAGTATGGAGAGGC | | | |
| 27 | rs10775648-F2 | CGCTCTTCCGATCTCTGCTTCGAGTCTCTGCATCGCG | HUNK | 21 | rs10775648 |
| 28 | rs10775648-R2 | CGCTCTTCCGATCTGACGCAAAGGAGTATGGAGAGGC | | | |
| 29 | rs2073370-F | CGCTCTTCCGATCTCTGGCCAGACATGTGCCTCGAAG | TCP10L | 21 | rs2073370 |
| 30 | rs2073370-R | CGCTCTTCCGATCTGACTCGTGCAGCAGAAGACACTT | | | |
| 31 | rs457705-F | CGCTCTTCCGATCTCTGGCCCAAGACCAACTGTGTCC | DONSON | 21 | rs457705 |
| 32 | rs457705-R | CGCTCTTCCGATCTGACCAAGGACGACTGGCTGTTCC | | | |
| 33 | COL18A1-F | CGCTCTTCCGATCTCTGCAGCAAAGCCACAGTCCTTC | COL18A1 | X | - |
| 34 | COL18A1-R | CGCTCTTCCGATCTGACCCCCACCCTGACATCCTTAC | | | |
| 35 | TBL1X-F | CGCTCTTCCGATCTCTGCTGTTTTGGGTGGGTTTGGT | TBL1X | X | - |
| 36 | TBL1X-R | CGCTCTTCCGATCTGACATTTGAAAGTGGGGCACCAT | | | |
| 37 | CTPS2-F | CGCTCTTCCGATCTCTGGGTACTTCTGGGATGCCTTG | CTPS2 | X | - |
| 38 | CTPS2-R | CGCTCTTCCGATCTGACGTTGCAGACTAAGGCCCACC | | | |
| 39 | PHF16-F | CGCTCTTCCGATCTCTGTGTTGGTCTTGTTCTTGCGT | PHF16 | X | - |
| 40 | PHF16-R | CGCTCTTCCGATCTGACGACCCATCTGTGACGCTGTG | | | |
| 41 | ELK1-F | CGCTCTTCCGATCTCTGGGGCCCAGGGAGTCATACAT | ELK1 | X | - |
| 42 | ELK1-R | CGCTCTTCCGATCTGACTCACGGCCTTCTTCTCCAGG | | | |
| 43 | TMEM185A-F | CGCTCTTCCGATCTGACACACACTGCCCATGGAAC | TMEM185A | Y | - |
| 44 | TMEN185A-R | CGCTCTTCCGATCTGACCAACTCAGCCAAGCCCAGTC | | | |
| 45 | CY_2655422-674_S-F | CGCTCTTCCGATCTCTGAGACCACACGATGAATGCGT | SRY | Y | - |
| 46 | CY_2655422-674_S-R | CGCTCTTCCGATCTGACGGCAACGTCCAGGATAGAGT | | | |

Next, in order to perform a sequence reaction using Miseq, an index sequence for identifying a sample, and P5 and P7 for binding a flow cell were added to both terminals of the multiplex PCR amplification product.

PCR was performed using a Multiplex PCR Assay kit using 1.25 μM of each of D501-F (SEQ ID No: 47), D701-R (SEQ ID No: 48), D702-R (SEQ ID No: 49), D703-R (SEQ ID No: 50), D704-R (SEQ ID No: 51), D705-R (SEQ ID No: 52), and D706-R (SEQ ID No: 53), which were shown in Table 3, as primers.

In the reaction, after modification performed for 3 minutes at 94°C, a cycle of 45 seconds at 94°C, 60 seconds at 50°C, and 30 seconds at 72°C was performed 5 times as PCR conditions. Then, the reaction was performed such that a cycle of 45 seconds at 94°C, 60 seconds at 55°C, and 30 seconds at 72°C was performed 11 times.

The obtained PCR product was purified using AMPure XP Kit (manufactured by BECKMAN COULTER), and the concentration of the PCR product was measured using Bioanalyzer.

Quantitative determination was performed using KAPA Library Quantification Kits manufactured by NIPPON Genetics Co, Ltd as more accurate quantitative determination of the amplification product.

[Table 3]

| SEQ ID No. | Primer name | Base sequences (5'→ 3') |
|---|---|---|
| 47 | D501-F | AATGATACGGCGACCACCGAGATCTACACTATAGCCTTCTTTCCCTACACGACGCTCTTCCGATCTCTG |
| 48 | D701-R | LAAGCAGAAGACGGCATACGAGATCGAGTAATGTGACTGGAGTTCAGACGTGTGCTCTTCCGATCTGAC |
| 49 | D702-R | CAAGCAGAAGACGGCATACGAGATTCTCCGGAGTGACTGGAGTTCAGACGTGTGCTCTTCCGATCTGAC |
| 50 | D703-R | CAAGCAGAAGACGGCATACGAGATAATGAGCGGTGACTGGAGTTCAGACGTGTGCTCTTCCGATCTGAC |
| 51 | D704-R | CAAGCAGAAGACGGCATACGAGATGGAATCTCGTGACTGGAGTTCAGACGTGTGCTCTTCCGATCTGAC |
| 52 | D705-R | CAAGCAGAAGACGGCATACGAGATTTCTGAATGTGACTGGAGTTCAGACGTGTGCTCTTCCGATCTGAC |
| 53 | D706-R | CAAGCAGAAGACGGCATACGAGATACGAATTCGTGACTGGAGTTCAGACGTGTGCTCTTCCGATCTGAC |

18

(Sequence Analysis of Amplification Product)

Analysis was performed such that 23 types of mixed multiplex PCR amplification products were sequenced using v2 300 Cycle (manufactured by Illumina, Inc.), the obtained FastQ file was mapped to a human genome sequence (hg19) using BWA, genetic polymorphism information was extracted using SAMtools, and the number of sequence leads of each detection region were calculated using BEDtools.

(Genotype Analysis)

It was possible to confirm that three cells out of 20 cells had SNP information different from the others by comparing SNPs of gene regions, which were shown in Tables 1 and 2, of chromosome 13, chromosome 18, and chromosome 21 with each other using a genome analyzer Miseq manufactured by Illumina, Inc., using whole genome amplification products amplified from each cell. In addition, it was possible to further confirm that two cells had SNP information different from the others. A cell which was expected to be a white blood cell in accordance with the shape of a nucleus was separately collected from the top of a slide, which was coated with blood, using a micromanipulator, and DNA was amplified similarly to 20 cells to check SNP. As a result, it was confirmed that SNP of the cell was coincident with SNP of the 15 cells. From the above, it was confirmed that five cells were fetus-derived nucleated red blood cells and were defined as dizygotic twin fetuses since the SNP information of the three cells and the SNP information of the two cells which were different from each other were obtained, and the 15 cells were mother-derived nucleated cells.

(Detection of Numerical Abnormality)

The amount of amplification product of the detection region of chromosome 21 of a nucleated red blood cell which was identified as being derived from a fetus and was discriminated through sequence analysis of a multiplex PCR amplification product was defined using Miseq. The amount of amplification product of a detection region of chromosome 21 of one cell out of nucleated cells which were identified as being derived from a mother was separately defined using a genome analyzer Miseq manufactured by Illumina, Inc.. The proportions of the amounts of these two amplification products were calculated. As a result, it was assumed that the proportions were values close to 1:1 and twin fetuses had a normal number of chromosomes.

[Example 2]

It was confirmed that membrane properties of a subject of a pregnant woman different from the pregnant woman whose blood was collected in Example 1 were dichorionic diamniotic twin fetuses, through an ultrasonic inspection. The genders of the fetuses were a male fetus and a female fetus. At this point in time, it was confirmed that the fetuses were dizygotic twin fetuses. It was necessary to obtain nucleated red blood cells having three types of genotypes, which are derived from a mother, a male fetus, and a female fetus, as genotypes of nucleated red blood cells, based on the results of the genders. Therefore, collecting 20 or more cells was aimed for isolation of candidate cells of nucleated red blood cells.
Similarly to Example 1, 20 candidate cells of nucleated red blood cells were collected in a sorting step of fetal nucleated red blood cells, and the process was similarly performed up to a DNA amplification step. In a multiplex PCR step after this, it was confirmed that there were four fetus-derived nucleated red blood cells through checking SNPs in the same manner as in Example 1. Since fetuses are a male fetus and a female fetus, the fetus-derived nucleated red blood cells were discriminated through checking the presence or absence of sex chromosomes. As a result, it was confirmed that there are two male fetus-derived nucleated red blood cells and two female fetus-derived nucleated red blood cells.
When the numerical abnormality of the chromosomes of the fetuses, it was assumed that the twin fetuses had a normal number of chromosomes.

[Example 3]

Peripheral blood was further collected from a pregnant woman different from the pregnant women whose blood was collected in Examples 1 and 2, and it was confirmed that membrane properties of the subject were dichorionic diamniotic twin fetuses, through an ultrasonic inspection. The genders of the fetuses were a male fetus and a female fetus. At this point in time, it was confirmed that the fetuses were dizygotic twin fetuses. It was necessary to obtain nucleated red blood cells having three types of genotypes, which were derived from a mother, a male fetus, and a female fetus, as genotypes of nucleated red blood cells, based on the results of the genders. Therefore, collection of 10 cells was performed for isolation of nucleated red blood cells in order to check the effectiveness of the present invention.
Similarly to Example 2, 10 cells were collected in a sorting step of fetal nucleated red blood cells, and the process was similarly performed up to a DNA amplification step. In a multiplex PCR step after this, it was confirmed that there were four fetus-derived nucleated red blood cells through checking SNPs in the same manner as in Example 2. However, it became clear that it was impossible to obtain only male fetus-derived nucleated red blood cells from fetuses and to obtain female fetus-derived nucleated red blood cells.

[Sequence Table]

International Application W-5427PCT Noninvasive Discrimination Method of Chromosomal Heteroploidy of Fetus Based on International Reception Patent Cooperation Treaty JP15077328 20150928----00050038751501930517 Normal 201509281543282015072209584422790_P1AP101_W-3.app

## Claims

1. A noninvasive discrimination method of chromosomal heteroploidy of a fetus, the method comprising:

   a step of analyzing DNA of a candidate cell of a nucleated red blood cell isolated from maternal blood, wherein the fetuses are fetuses classified into any one case selected from monotocous, monochorionic monoamniotic twin fetuses, monochorionic diamniotic twin fetuses, and dichorionic diamniotic twin fetuses based on results of ultrasonic inspections and the number of candidate cells of nucleated red blood cells to be used for analyzing the DNA is optimized based on the classification.

2. The noninvasive discrimination method of chromosomal heteroploidy of a fetus according to claim 1, wherein the optimization includes setting of the number of candidate cells of nucleated red blood cells used for analyzing the DNA as number of cases of monotocous ≤ number of cases of monochorionic monoamniotic twin fetuses < number of cases of monochorionic diamniotic twin fetuses < number of cases of dichorionic diamniotic twin fetuses.

3. The noninvasive discrimination method of chromosomal heteroploidy of a fetus according to claim 1 or 2, wherein the step of analyzing DNA of a candidate cell of a nucleated red blood cell isolated from maternal blood includes the following steps:

   <step 1> a step of acquiring classification information of fetuses based on the membranous information obtained through an ultrasonic inspection, the classification information being shown in the following a) to d),

a) monotocous,
b) monochorionic monoamniotic twin fetuses,
c) monochorionic diamniotic twin fetuses, and
d) dichorionic diamniotic twin fetuses,

<step 2> a step of determining the number of candidate cells of nucleated red blood cells isolated from a maternal blood sample, based on the classification information,
<step 3> a step of isolating the number of candidate cells of nucleated red blood cells from the maternal blood sample,
<step 4> a step of performing whole genome amplification on each of the isolated candidate cells of nucleated red blood cells,
<step 5> a step of performing genetic polymorphism analysis and/or a Y chromosome existence check, using whole genome amplification product,
<step 6> a step of comparing the number of genotypes of fetus-derived nucleated red blood cells detected through the genetic polymorphism analysis and/or the Y chromosome existence check with the number of genotypes of fetuses estimated from the classification information of the fetuses and selecting a process to proceed to the next step in a case where both numbers of genotypes are coincident with each other or a process to return to step 3 in a case where both numbers of genotypes are not coincident with each other, and
<step 7> a step of discriminating chromosomal heteroploidy of the fetus-derived nucleated red blood cells.

4. A noninvasive discrimination system of chromosomal heteroploidy of a fetus, the system comprising:

fetus information acquisition means for acquiring classification information of fetuses based on results of ultrasonic inspections;
form information acquisition means for acquiring form information of cells contained in a maternal blood sample, based on the classification information of fetuses;
cell isolation means for isolating the cells contained in the maternal blood sample,
a PCR device of performing whole genome amplification on the isolated cells;
a multiplex PCR device of performing multiplex PCR on the whole genome amplification products; and
a DNA sequencing device of decoding a base sequence of the PCR amplification product and measuring the number of sequence leads.

5. The noninvasive discrimination system of chromosomal heteroploidy of a fetus according to claim 4,
wherein the number of candidate cells of nucleated red blood cells to be isolated is determined based on the information acquired by the fetus information acquisition means,
wherein the candidate cells of nucleated red blood cells to be isolated are selected based on the information acquired by the form information acquisition means,
wherein the candidate cells of nucleated red blood cells are isolated by the isolation means,
wherein whole genome amplification is performed on the candidate cells of nucleated red blood cells isolated using the PCR device,
wherein multiplex PCR is performed using the multiplex PCR device, and
wherein measurement of the number of sequence leads and decoding of base sequences of DNA fragments obtained through multiplex PCR using the DNA sequencing device are performed, the number of genotypes of candidate cells of nucleated red blood cells detected through genetic polymorphism analysis and/or a Y chromosome existence check with the number of genotypes of fetuses estimated from classification of fetuses, the process proceeds to the next step in a case where both numbers of genotypes are coincident with each other, and the process returns to the step of isolating the candidate cells of nucleated red blood cells in a case where both numbers of genotypes are not coincident with each other to repeat the steps thereafter.

## FIG. 1

START

↓

OBTAIN CLASSIFICATION INFORMATION OF FETUS BASED ON MEMBRANOUS INFORMATION OBTAINED THROUGH ULTRASONIC INSPECTION

↓

DETERMINE NUMBER OF CANDIDATE CELLS OF NUCLEATED RED BLOOD CELLS TO BE ISOLATED

↓

ISOLATE CANDIDATE CELLS OF NUCLEATED RED BLOOD CELLS FROM MATERNAL BLOOD SAMPLE

↓

WHOLE GENOME AMPLIFICATION

↓

GENETIC POLYMORPHISM ANALYSIS / Y CHROMOSOME EXISTENCE CHECK

↓

COMPARE NUMBER OF GENOTYPES OF NUCLEATED RED BLOOD CELLS DETECTED WITH NUMBER OF GENOTYPES OF FETUSES ESTIMATED

↓

IS NUMBER OF GENOTYPES OF NUCLEATED RED BLOOD CELLS DETECTED COINCIDENT WITH NUMBER OF GENOTYPES OF FETUSES ESTIMATED?

— BEING NOT COINCIDENT

↓ BEING COINCIDENT

DISCRIMINATION OF CHROMOSOMAL HETEROPLOIDY

↓

END

### INTERNATIONAL SEARCH REPORT

| International application No. |
| --- |
| PCT/JP2015/077328 |

**A. CLASSIFICATION OF SUBJECT MATTER**
*C12Q1/68*(2006.01)i, *C12M1/34*(2006.01)i, *C12N15/09*(2006.01)i

According to International Patent Classification (IPC) or to both national classification and IPC

**B. FIELDS SEARCHED**

Minimum documentation searched (classification system followed by classification symbols)
C12Q1/68, C12N15/00-15/90

Documentation searched other than minimum documentation to the extent that such documents are included in the fields searched
Jitsuyo Shinan Koho          1922-1996   Jitsuyo Shinan Toroku Koho   1996-2015
Kokai Jitsuyo Shinan Koho    1971-2015   Toroku Jitsuyo Shinan Koho   1994-2015

Electronic data base consulted during the international search (name of data base and, where practicable, search terms used)
JSTPlus/JMEDPlus/JST7580(JDreamIII),
CAplus/MEDLINE/EMBASE/BIOSIS/WPIDS(STN)

**C. DOCUMENTS CONSIDERED TO BE RELEVANT**

| Category* | Citation of document, with indication, where appropriate, of the relevant passages | Relevant to claim No. |
| --- | --- | --- |
| A | XU Z et al., Non-Invasive Prenatal Diagnosis: A Comparison of Cell Free Fetal DNA (cffDNA) Based Screening and Fetal Nucleated Red Blood Cell (fnRBC) Initiated Testing, North American Journal of Medicine and Science, 2013, 6(4), pp.194-199, ISSN: 1944-0944 | 1-5 |
| A | Kiyotake ICHIZUKA et al., "Tatai no Makusei Shindan", Clinical Gynecology and Obstetrics, 2008 Nen, vol.62, no.3, pages 265 to 267, ISSN: 0386-9865 | 1-5 |
| A | SEPPO A et al., Detection of circulating fetal cells utilizing automated microscopy: potential for noninvasive prenatal diagnosis of chromosomal aneuploidies, Prenat. Diagn., 2008, 28(9), pp.815-821, ISSN: 1097-0223 | 1-5 |

☒ Further documents are listed in the continuation of Box C.     ☐ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| 14 December 2015 (14.12.15) | 28 December 2015 (28.12.15) |

| Name and mailing address of the ISA/ | Authorized officer |
| --- | --- |
| Japan Patent Office 3-4-3,Kasumigaseki,Chiyoda-ku, Tokyo 100-8915,Japan | Telephone No. |

Form PCT/ISA/210 (second sheet) (July 2009)

**INTERNATIONAL SEARCH REPORT**

International application No.

PCT/JP2015/077328

C (Continuation). DOCUMENTS CONSIDERED TO BE RELEVANT

| Category* | Citation of document, with indication, where appropriate, of the relevant passages | Relevant to claim No. |
|---|---|---|
| A | MAVROU A et al., Identification of nucleated red blood cells in maternal circulation: a second step in screening for fetal aneuploidies and pregnancy complications, Prenat. Diagn., 2007, 27(2), pp.150-153, ISSN: 1097-0223 | 1-5 |
| A | HENNERBICHLER S et al., Fetal nucleated red blood cells in peripheral blood of pregnant women: detection and determination of location on a slide using laser-scanning cytometry, Prenat. Diagn., 2003, 23(9), pp.710-715, ISSN: 1097-0223 | 1-5 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004533243 A
- JP 2004531271 A
- JP 2012513217 A
- WO 2012023298 A
- WO 2012166425 A